# EUROPÄISCHE PATENTANMELDUNG

(11) **EP 4 285 844 A1**
(43) Veröffentlichungstag der Anmeldung: **06.12.2023**
(21) Anmeldenummer: 23175951.5
(22) Anmeldetag: 30.05.2023
(51) Int. Cl.: A61B 17/15

(54) **CHIRURGISCHES INSTRUMENT**

(30) Priorität: 03.06.2022 DE 102022205693
(71) Anmelder: Aesculap AG, 78532 Tuttlingen (DE)
(72) Erfinder: Firmbach, Franz-Peter, 78576 Emmingen-Liptingen (DE); Anhorn, Svenja, 72535 Heroldstatt (DE)
(74) Vertreter: Patentanwälte Ruff, Wilhelm, Beier, Dauster & Partner mbB

(57) **Zusammenfassung**

Die Erfindung betrifft ein chirurgisches Instrument zur Verwendung bei einer Kniegelenkersatzoperation, aufweisend einen Grundkörper, welcher eingerichtet ist zur lösbaren Befestigung an einem Tibiaschnittblock zur Schnittführung an einer proximalen Tibia, einen ersten Taster und einen zweiten Taster, welche jeweils relativ zu dem Grundkörper beweglich an demselben gelagert und zwischen einem ersten Ende und einem zweiten Ende längserstreckt sind, wobei die ersten Enden jeweils eine Referenzfläche aufweisen, welche zur Kontaktierung eines Tibiaplateaus der Tibia entlang einer Kontaktrichtung eingerichtet ist, wobei wenigstens ein zur wahlweisen Anbringung an einem der beiden Taster vorgesehenes Kompensationselement vorhanden ist und einen Steckabschnitt sowie eine Kontaktfläche aufweist, und wobei die ersten Enden jeweils einen komplementären Steckabschnitt aufweisen, welcher zur form- und/oder kraftschlüssigen lösbaren Verbindung mit dem Steckabschnitt des Kompensationselements eingerichtet ist, und wobei die Kontaktfläche - in einem an dem jeweiligen Taster angebrachten Zustand des Kompensationselements - zur Kontaktierung des Tibiaplateaus anstelle der jeweiligen Referenzfläche eingerichtet und entlang der Kontaktrichtung um ein Dickenmaß beabstandet von der jeweiligen Referenzfläche angeordnet ist.

## Beschreibung

Die Erfindung betrifft ein chirurgisches Instrument zur Verwendung bei einer Kniegelenkersatzoperation.

Bei einer Kniegelenkersatzoperation (englisch: total knee arthroplasty (TKA)) werden abgenutzte oder anderweitig durch Krankheit oder einen Unfall beeinträchtigte Gelenkflächen des Femurs und/oder der Tibia durch künstliche Gelenkflächen einer Kniegelenkprothese ersetzt. Solche Kniegelenkprothesen umfassen üblicherweise eine Femurkomponente und eine Tibiakomponente. Die Femurkomponente wird am distalen Ende des Femurs implantiert. Die Tibiakomponente wird am proximalen Ende der Tibia implantiert.

Vor der Implantation der prothetischen Komponenten werden der distale Femur und die proximale Tibia reseziert. Hierfür bringt der Chirurg unterschiedliche Resektionsschnitte an und trennt Knochen- und/oder Knorpelmaterial von dem jeweiligen Knochen ab. Durch die Resektion wird der jeweilige Knochen in seiner Form an die aufzunehmende prothetische Komponente angepasst.

Die Resektion kann auf der Grundlage unterschiedlicher Konzepte ausgeführt werden. Ein Konzept zielt darauf ab, die Spannungen der Bänder des Knies bei der Gelenkbewegung ausgeglichen zu halten. Hierdurch soll eine bessere Funktion der Kniegelenkprothese gewährleistet werden. Dieses Konzept wird allgemein als "gap balancing" bezeichnet. Bei anderen Konzepten entfernt der Chirurg mittels der Resektion eine bestimmte Menge an Knochen- und Knorpelmaterial. Solche Konzepte werden allgemein als "measured resection" bezeichnet. Die Ausrichtung der Resektionsschnitte in Bezug auf die Anatomie des Patienten bestimmt die spätere Ausrichtung der implantierten Komponenten und folglich auch die Orientierung der prothetischen Gelenkachsen. Die Ausrichtung der Resektionsschnitte ist daher von besonderer Bedeutung.

Bei der Ausrichtung der Resektionsschnitte werden in erster Linie drei Ansätze unterschieden: mechanisch, anatomisch und kinematisch. Bei der mechanischen Ausrichtung wird die proximale Tibia senkrecht zur Längsachse des Tibiaschafts reseziert. Die Resektion des distalen Femurs erfolgt entsprechend hieran angepasst. Erforderlichenfalls werden Bandentlastungen (englisch: ligament releases) durchgeführt. Bei der anatomischen Ausrichtung wird versucht, die Tibia in einem Varus-Winkel von 3° zu resezieren. Die Femurresektion und Bandentlastungen werden mit dem Ziel einer geraden Hüft-Knie-Knöchel-Achse des Beins durchgeführt. Das Ziel der kinematischen Ausrichtung (englisch: kinematic alignment, nachfolgend abgekürzt als KA besteht darin, die künstlichen Gelenkflächen der prothetischen Komponenten auf dem Niveau der präarthritischen, defektfreien natürlichen Gelenkflächen zu implantieren. Klinische Studien haben gezeigt, dass mittels KA oftmals eine verbesserte Patientenzufriedenheit erreicht werden kann, da die Funktion des künstlichen Kniegelenks als eher natürlich empfunden wird.

Vor diesem Hintergrund besteht ein grundsätzlicher Bedarf nach möglichst präzisen, einfach verwendbaren und kosteneffizienten chirurgischen Instrumenten zur Umsetzung des KA.

Die vorliegende Erfindung befasst sich mit einem solchen chirurgischen Instrument. Im Speziellen befasst sich die Erfindung mit einem chirurgischen Instrument zur Ausrichtung der Tibiaresektion. Solche Instrumente werden auch als (tibiales) Ausrichtinstrument oder tibial resection guide device bezeichnet. Aus dem Stand der Technik sind unterschiedliche derartige chirurgische Instrumente bekannt.

Aus der WO 2020/049421 A1 ist ein tibiales Ausrichtinstrument mit einem Grundkörper und zwei Tastern bekannt. Der Grundkörper ist in Form eines Zapfens gestaltet und an einem hierfür eingerichteten Abschnitt eines Tibiaschnittblocks befestigbar. Die beiden Taster sind jeweils relativ zu dem Grundkörper beweglich an demselben gelagert und zwischen einem ersten Ende und einem zweiten Ende längserstreckt. Die ersten Enden sind jeweils zur Kontaktierung der proximalen Tibia, genauer: des Tibiaplateaus, eingerichtet. Die beiden Taster können jeweils relativ zu dem Grundkörper in unterschiedlichen proximodistalen Positionen angebracht werden. Hierdurch sollen Knochen- und/oder Knorpeldefekte an dem Tibiaplateau maßlich ausgeglichen werden.

Aus der US 2019/0231365 A1 ist ein weiteres chirurgisches Instrument zur Ausrichtung des Tibiaschnitts bekannt. Die ersten Enden der beiden Taster dieses Instruments sind mittels eines Einstellmechanismus in ihrer Position relativ zu dem Tibiaplateau einstellbar. Der Einstellmechanismus umfasst jeweils eine Linearführung mit einer Art Ratsche an jedem der Taster. Der Aufbau ist vergleichsweise komplex.

Aufgabe der Erfindung ist es, ein chirurgisches Instrument zur Verwendung bei einer Kniegelenkersatzoperation bereitzustellen, das Vorteile gegenüber dem Stand der Technik bietet und insbesondere eine verbesserte Ausrichtung des proximalen Tibiaschnitts ermöglicht, wobei gleichzeitig ein einfacher Aufbau erreicht oder beibehalten werden soll.

Diese Aufgabe wird durch das Bereitstellen eines chirurgischen Instruments mit den Merkmalen des Anspruchs 1 gelöst.

Das erfindungsgemäße chirurgische Instrument weist auf: einen Grundkörper, welcher eingerichtet ist zur lösbaren Befestigung an einem Tibiaschnittblock zur Schnittführung an einer proximalen Tibia, einen ersten Taster und einen zweiten Taster, welche jeweils relativ zu dem Grundkörper beweglich an demselben gelagert und zwischen einem ersten Ende und einem zweiten Ende längserstreckt sind, wobei die ersten Enden jeweils eine Referenzfläche aufweisen, welche zur Kontaktierung eines Tibiaplateaus der Tibia entlang einer Kontaktrichtung eingerichtet ist, wobei wenigstens ein zur wahlweisen Anbringung an einem der beiden Taster vorgesehenes Kompensationselement vorhanden ist und einen Steckabschnitt sowie eine Kontaktfläche aufweist, wobei die ersten Enden jeweils einen komplementären Steckabschnitt aufweisen, welcher zur form- und/oder kraftschlüssigen lösbaren Verbindung mit dem Steckabschnitt des Kompensationselements eingerichtet ist, und wobei die Kontaktfläche - in einem an dem jeweiligen Taster angebrachten Zustand des Kompensationselements - zur Kontaktierung des Tibiaplateaus anstelle der jeweiligen Referenzfläche eingerichtet und entlang der Kontaktrichtung um ein Dickenmaß beabstandet von der jeweiligen Referenzfläche angeordnet ist. Durch die erfindungsgemäße Lösung können Defekte des Tibiaplateaus auf einfache Weise maßlich ausgeglichen werden. Zu diesem Zweck ist das wenigstens eine Kompensationselement vorhanden und wahlweise an dem ersten Taster oder dem zweiten Taster, genauer: an deren ersten Enden, anbringbar. Im Vergleich zu aus dem Stand der Technik bekannten Lösungen, bei welchen der Ausgleich über einen Einstellmechanismus oder eine Positionsänderung der Taster erfolgt, ist die erfindungsgemäße Lösung insbesondere weniger fehleranfällig. Denn der Chirurg kann einfach und unmissverständlich erkennen, ob eine Kompensation vorliegt oder nicht. Ist das wenigstens eine Kompensationselement an einem der beiden Taster angebracht, liegt eine Kompensation vor. Ist dies nicht der Fall, liegt keine Kompensation vor. Bei den aus dem Stand der Technik bekannten Lösungen kann es zu schwer erkennbaren Fehleinstellungen kommen. Zudem weist das erfindungsgemäße chirurgische Instrument einen einfachen Aufbau auf. Auch dies im Gegensatz zu bekannten Lösungen mit einer Einstellbarkeit der Position der ersten Enden. Der einfache Aufbau erlaubt zudem eine kosteneffiziente Montage sowie Demontage. Letzteres insbesondere zu Reinigungszwecken. Auch die Reinigung an sich wird durch den einfachen Aufbau erleichtert. Das wenigstens eine Kompensationselement ist wahlweise mit dem ersten Ende des ersten Tasters oder dem ersten Ende des zweiten Tasters zusammensteckbar. Zu diesem Zweck weist das wenigstens eine Kompensationselement den besagten Steckabschnitt auf. Die beiden ersten Enden weisen jeweils einen komplementären Steckabschnitt auf. In angebrachtem Zustand bilden der Steckabschnitt und der jeweilige komplementäre Steckabschnitt eine lösbare Steckverbindung. Die Steckverbindung ist ohne Zuhilfenahme von Werkzeug oder sonstigen Hilfsmitteln rein manuell etablierbar. Die Steckverbindung ist unmittelbar zwischen dem Kompensationselement und dem jeweiligen ersten Ende ausgebildet. Zusätzliche Verbindungsmittel sind nicht vorhanden. In einem Zustand des chirurgischen Instruments, in welchem das wenigstens eine Kompensationselement an keinem der Taster angebracht ist, liegen deren Referenzflächen jeweils an dem Tibiaplateau an. Beispielsweise liegt die Referenzfläche des ersten Tasters an dem medialen Tibiaplateau an und die Referenzfläche des zweiten Tasters liegt an dem lateralen Tibiaplateau an. In einem Zustand des chirurgischen Instruments, in welchem das wenigstens eine Kompensationselement an dem ersten Taster angebracht ist, kontaktiert die Kontaktfläche des Kompensationselements das (mediale) Tibiaplateau. Der Kontakt erfolgt anstelle der Referenzfläche des ersten Tasters. Mit anderen Worten ausgedrückt überdeckt die Kontaktfläche die Referenzfläche und ist hierbei um das besagte Dickenmaß von derselben beabstandet. Das Dickenmaß ist an den zu kompensierenden Defekt angepasst. Bei einer bevorzugten Ausgestaltung umfasst das chirurgische Instrument mehrere unterschiedliche Kompensationselemente zur Kompensation unterschiedlich stark ausgeprägter Defekte an dem Tibiaplateau. Bei einer bevorzugten Ausgestaltung sind die Referenzflächen der beiden ersten Enden in einer gemeinsamen Referenzebene angeordnet. Die Referenzflächen können jeweils tatsächliche Flächen, Linien oder Punkte sein. Letzteres beispielsweise dann, wenn die ersten Enden angespitzt sind, so dass auch von einer ersten Tasterspitze und einer zweiten Tasterspitze gesprochen werden kann. Vorzugsweise sind die Referenzflächen durch eine dem Tibiaplateau zugewandte, bevorzugt plane, Vorderseite des jeweiligen ersten Endes gebildet. Die Kontaktfläche ist bei unterschiedlichen Ausgestaltungen unterschiedlich und beispielsweise plan, kegelförmig oder konkav.

Die in dieser Beschreibung verwendeten Lage- und Richtungsbezeichnungen beziehen sich auf den Körper eines Patienten, insbesondere dessen Tibia, und sind insoweit gemäß ihrer üblichen anatomischen Bedeutung zu verstehen. Folglich bedeutet "anterior" vordere(r) oder vorne liegend, "posterior" hintere(r) oder hinten liegend, "medial" innere(r) oder innen liegend, "lateral" äußere(r) oder außen liegend, "proximal" zum Körperzentrum hin und "distal" vom Körperzentrum entfernt. Weiter bedeuten "proximodistal" entlang, vorzugsweise parallel zu, einer proximal-distal ausgerichteten Achse, "anteroposterior" entlang, vorzugsweise parallel zu, einer anterior-posterior ausgerichteten Achse und "mediolateral" entlang, vorzugsweise parallel zu, einer medial-lateral ausgerichteten Achse. Die besagten Achsen sind orthogonal zueinander ausgerichtet und können selbstverständlich in Bezug zu nicht mit der Anatomie des Patienten in Verbindung stehenden X-, Y- und Z-Achsen gesetzt werden. Beispielsweise kann die proximaldistale Achse alternativ als X-Achse bezeichnet werden. Die medial-laterale Achse kann als Y-Achse bezeichnet werden. Die anterior-posteriore Achse kann als Z-Achse bezeichnet werden. Zur verbesserten Veranschaulichung und der Einfachheit der Bezeichnungen wegen werden nachfolgend in erster Linie die besagten anatomischen Lage- und Richtungsbezeichnungen verwendet. Weiter werden Bezeichnungen wie "Rückseite" einer Komponente oder eines Abschnitts des chirurgischen Instruments, beispielsweise des Kompensationselements, in Bezug auf eine distal gerichtete Blickrichtung verwendet. Dementgegen werden Bezeichnungen wie "Vorderseite" in Bezug auf eine proximal gerichtete Blickrichtung verwendet.

In Ausgestaltung der Erfindung ist der Steckabschnitt entlang einer Steckrichtung mit dem jeweiligen komplementären Steckabschnitt zusammensteckbar, welche parallel zu einer Längsachse des jeweiligen Tasters und/oder orthogonal zu der Kontaktrichtung orientiert ist. Eine solche Orientierung der Steckrichtung erlaubt insbesondere ein ergonomisches Anbringen und Abnehmen des wenigstens einen Kompensationselements. In der Verwendung des chirurgischen Instruments sind die Längsachsen der Taster wenigstens im Wesentlichen anterioposterior orientiert. Die Kontaktrichtung ist wenigstens im Wesentlichen distal orientiert. Durch die orthogonal zu der Kontaktrichtung orientierte Steckrichtung können der Steckabschnitt und der jeweilige komplementäre Steckabschnitt auch dann zusammengesteckt (und auseinandergesteckt) werden, wenn die beiden Taster bereits im Bereich des Tibiaplateaus positioniert sind.

In weiterer Ausgestaltung der Erfindung weisen der Steckabschnitt und/oder die komplementären Steckabschnitte jeweils einen rotationsunsymmetrischen Querschnitt auf. Der rotationsunsymmetrische Querschnitt wirkt einer ungewollten relativen Rotation zwischen dem Steckabschnitt und dem betreffenden komplementären Steckabschnitt entgegen. Die Rotationssymmetrie bezieht sich hierbei auf die Steckrichtung, entlang derer der Steckabschnitt mit dem jeweiligen komplementären Steckabschnitt zusammensteckbar ist.

In weiterer Ausgestaltung der Erfindung weisen die komplementären Steckabschnitte jeweils einen längserstreckten Aufnahmeschlitz auf, welcher zur kraft- und/oder formschlüssigen Aufnahme des Steckabschnitts eingerichtet ist. Der Steckabschnitt ist wenigstens abschnittsweise komplementär zu dem Aufnahmeschlitz gestaltet. Bei einer Ausgestaltung ist der Steckabschnitt des Kompensationselements entlang der Längserstreckung des Aufnahmeschlitzes in denselben einsteckbar. Bei einer weiteren Ausgestaltung ist der Steckabschnitt quer, vorzugsweise orthogonal, zur Längserstreckung des Aufnahmeschlitzes in denselben einsteckbar. Der Aufnahmeschlitz ist vergleichsweise einfach zu fertigen und ermöglicht zudem eine besonders einfache und gründliche Reinigung. Vorzugsweise ist der Aufnahmeschlitz gerade längserstreckt. Bei einer Ausgestaltung ist der Aufnahmeschlitz einends offen. Bei einer weiteren Ausgestaltung ist der Aufnahmeschlitz beidseits geschlossen. Denkbar ist zudem eine beidseits offene Gestaltung. Zudem kann der Schlitz in seiner Hochrichtung nach Art einer Nut einseitig durch einen Boden begrenzt sein. Alternativ kann der Aufnahmeschlitz entlang seiner Hochrichtung beidseits offen, d.h. als Durchgangsschlitz, gestaltet sein.

In weiterer Ausgestaltung der Erfindung ist der Aufnahmeschlitz entlang seiner Längsrichtung zwischen einer Einführöffnung und einem Anschlag längserstreckt. Folglich ist der Aufnahmeschlitz in Längsrichtung einends offen und andernends geschlossen. Die Einführöffnung ermöglicht ein ergonomisches Einstecken des Steckabschnitts in Längsrichtung des Aufnahmeschlitzes. Der Anschlag begrenzt eine Einstecktiefe des Steckabschnitts. Durch den Anschlag wird eine stets gleichbleibende Positionierung des Kompensationselements in Relation zu dem betreffenden Taster bei wiederholtem Anbringen und Abnehmen gewährleistet.

In weiterer Ausgestaltung der Erfindung reicht der Aufnahmeschlitz in einer quer zu seiner Längsrichtung orientierten Hochrichtung von der Referenzfläche bis auf eine Rückseite des jeweiligen ersten Endes, wobei die Rückseite der Referenzfläche entlang der Kontaktrichtung gegenüberliegt. In der Verwendung des chirurgischen Instruments ist die Kontaktrichtung wenigstens im Wesentlichen distal orientiert, d.h. die Referenzfläche des betreffenden ersten Endes liegt distal an dem Tibiaplateau an. Entsprechendes gilt sinngemäß für die Kontaktfläche des wenigstens einen Kompensationselements, sofern dieses an einem der beiden Taster angebracht ist. Die Rückseite liegt der Referenzfläche entlang der Kontaktrichtung, d.h. im Wesentlichen proximal, gegenüber. Die Hochrichtung des Aufnahmeschlitzes ist quer, vorzugsweise orthogonal, zu seiner Längsrichtung orientiert. In der Verwendung ist die Hochrichtung folglich proximodistal ausgerichtet. Bei dieser Ausgestaltung ist der Aufnahmeschlitz entlang seiner Hochrichtung durch das erste Ende erstreckt und reicht von der Rückseite bis auf die Referenzfläche. Eine solch durchgängige Gestaltung des Aufnahmeschlitzes erlaubt insbesondere eine nochmals vereinfachte Reinigung. Zudem erlaubt der insoweit durchgängige Aufnahmeschlitz eine verbesserte Einsehbarkeit des Tibiaplateaus, sofern das wenigstens eine Kompensationselement nicht an dem betreffenden ersten Ende angebracht ist. Die verbesserte Einsehbarkeit erlaubt eine präzise visuelle Kontrolle der zwischen der Referenzfläche und dem Tibiaplateau vorherrschenden Kontaktverhältnisse.

In weiterer Ausgestaltung der Erfindung ist der Aufnahmeschlitz zwischen zwei, vorzugsweise federelastischen, Zinkenabschnitten des jeweiligen ersten Endes gebildet, wobei die Zinkenabschnitte in einer quer zu der Längsrichtung des Aufnahmeschlitzes orientierten Querrichtung einander gegenüberliegen. Bei dieser Ausgestaltung der Erfindung weist das erste Ende folglich eine gabelförmige Gestaltung auf. Sofern die Zinkenabschnitte federelastisch gestaltet sind, unterstützt dies eine zuverlässige Verbindung zwischen dem Steckabschnitt und dem Aufnahmeschlitz.

In weiterer Ausgestaltung der Erfindung ragt der Steckabschnitt von einer Rückseite des Kompensationselements auf, welche der Kontaktfläche entlang der Kontaktrichtung gegenüberliegt. In der Verwendung des chirurgischen Instruments ist die Rückseite proximal orientiert. Vorzugsweise ist die Rückseite plan. Durch die Anordnung des Steckabschnitts auf der Rückseite ergibt sich eine verbesserte Einsehbarkeit. Dies ermöglicht eine besonders einfache visuelle Kontrolle der Steckverbindung.

In weiterer Ausgestaltung der Erfindung weist der Steckabschnitt einen Schaft und einen Kopf auf, wobei der Schaft zwischen der Rückseite des Kompensationselements und dem Kopf längserstreckt und quer zu seiner Längserstreckung mit dem komplementären Steckabschnitt des jeweiligen ersten Endes zusammensteckbar ist, und wobei der Kopf einen größeren Durchmesser als der Schaft aufweist. Vereinfacht ausgedrückt ist der Steckabschnitt bei dieser Ausgestaltung pilzkopfförmig und/oder als Pilzkopf gestaltet. Der Schaft ist einends mit der Rückseite und andernends mit dem Kopf verbunden. Der Schaft ist gerade längserstreckt. In der Verwendung des chirurgischen Instruments ist der Schaft proximodistal ausgerichtet und ragt proximal von der Rückseite auf. Der Kopf ist an einem proximalen Ende des Schafts angeordnet. Der Kopf bildet das Stirnende des Steckabschnitts. Zum Zusammenstecken mit dem jeweiligen komplementären Steckabschnitt wird der Schaft nicht etwa mit dem Kopf voran, sondern stattdessen quer, vorzugsweise orthogonal, zu seiner Längserstreckung mit dem komplementären Steckabschnitt zusammengeführt. Der Kopf fungiert als zusätzliches Halteelement und wirkt einer ungewollten Relativverlängerung des Schafts gegenüber dem komplementären Steckabschnitt entgegen. Zu diesem Zweck weist der Kopf einen größeren Durchmesser als der Schaft auf. Diese Ausgestaltung ist besonders vorteilhaft, wenn die komplementären Steckabschnitte der beiden Taster jeweils einen Aufnahmeschlitz aufweisen.

In weiterer Ausgestaltung der Erfindung weisen der Schaft und/oder der Kopf einen rotationsunsymmetrischen Querschnitt auf. Der rotationsunsymmetrische Querschnitt wirkt einer ungewollten relativen Rotation zwischen dem Steckabschnitt, insbesondere dem Schaft und/oder dem Kopf, und dem jeweiligen komplementären Steckabschnitt entgegen.

In weiterer Ausgestaltung der Erfindung ist die Kontaktfläche konvex gewölbt. Die konvexe Wölbung der Kontaktfläche erlaubt insbesondere eine verbesserte Einsehbarkeit der zwischen dem Kompensationselement und dem Tibiaplateau vorherrschenden Kontaktverhältnisse Dies erlaubt dem Chirurgen eine verbesserte visuelle Kontrolle.

In weiterer Ausgestaltung der Erfindung weist das wenigstens eine Kompensationselement einen Kugelsegmentabschnitt mit einer Kugelkalotte auf, welche die Kontaktfläche bildet. Der Kugelsegmentabschnitt ist an einer Vorderseite des Kompensationselements angeordnet. Die Kugelkalotte ist kreisförmig berandet. Dies gilt dementsprechend für die Kontaktfläche. Mit dieser Ausgestaltung gehen besondere Vorteile einher.

In weiterer Ausgestaltung der Erfindung sind mehrere unterschiedliche Kompensationselemente zur wahlweisen Anbringung an den beiden Tastern vorhanden, wobei die unterschiedlichen Kompensationselemente sich, vorzugsweise ausschließlich, unterscheiden im Hinblick auf ihr jeweiliges Dickenmaß und/oder einen Durchmesser der jeweiligen Kontaktfläche und/oder eine Wölbung der jeweiligen Kontaktfläche. Die unterschiedlichen Kompensationselemente ermöglichen die Kompensation unterschiedlich stark ausgeprägter kondylärer Defekte an dem Tibiaplateau. Je nach Ausprägung in distaler Richtung erfolgt die Kompensation mit einem vergleichsweise dicken oder dünnen Kompensationselement. Je nach mediolateraler und/oder anteroposteriorer Ausdehnung erfolgt die Kompensation mit einem vergleichsweise kleinen oder großen (Außen-)Durchmesser. Die unterschiedlichen Wölbungen erlauben eine besonders präzise Anpassung der Kompensation an die Geometrie des vorliegenden Defekts. Die unterschiedlichen Kompensationselemente sind hinsichtlich ihres jeweiligen Steckabschnitts identisch. Mit anderen Worten weisen die unterschiedlichen Kompensationselemente identisch gestaltete und/oder geformte Steckabschnitte auf. Hierdurch kann jedes der unterschiedlichen Kompensationselemente wahlweise an dem ersten Taster oder dem zweiten Taster angebracht werden.

Die Erfindung betrifft zudem ein chirurgisches Instrumentensystem zur Verwendung bei einer Kniegelenkersatzoperation mit einem chirurgischen Instrument nach einem der vorhergehenden Ansprüche, einem Tibiaschnittblock, an welchem das chirurgische Instrument lösbar befestigt ist, und mit einem extramedullären Ausrichtstab, welcher lösbar an dem Tibiaschnittblock befestigt ist.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt in schematischer Perspektivdarstellung eine Ausführungsform eines erfindungsgemäßen chirurgischen Instruments,
- Fig. 2: eine weitere schematische Perspektivdarstellung des chirurgischen Instruments nach Fig. 1 in einer exemplarischen intraoperativen Situation, in welcher das chirurgische Instrument an einer proximalen Tibia positioniert ist,
- Fig. 3: eine weitere schematische Perspektivdarstellung des chirurgischen Instruments nach den Fig. 1 und 2,
- Fig. 4: eine schematische Aufsicht des chirurgischen Instruments nach den Fig. 1 bis 3,
- Fig. 5: eine vergrößerte perspektivische Detaildarstellung des chirurgischen Instruments im Bereich eines Tasterendes, an welchem ein Kompensationselement lösbar angebracht ist,
- Fig. 6: eine weitere Darstellung des Detailbereichs nach Fig. 5, wobei das Kompensationselement von dem Tasterende gelöst ist,
- Fig. 7: in schematischer Perspektivdarstellung mehrere unterschiedliche Kompensationselemente des chirurgischen Instruments und
- Fig. 8: die unterschiedlichen Kompensationselemente nach Fig. 7 in schematischer Seitenansicht.

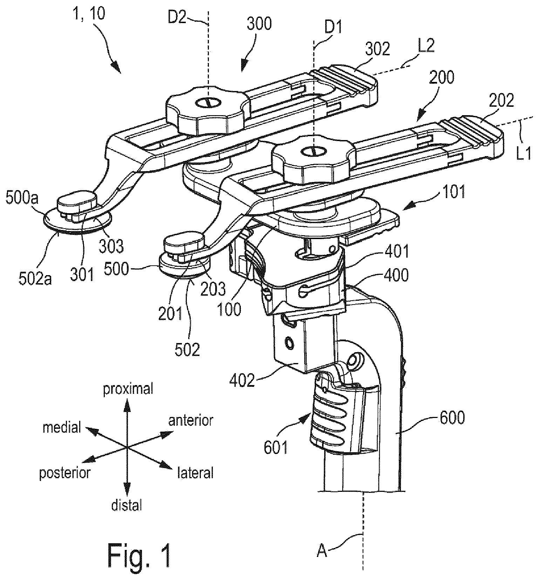

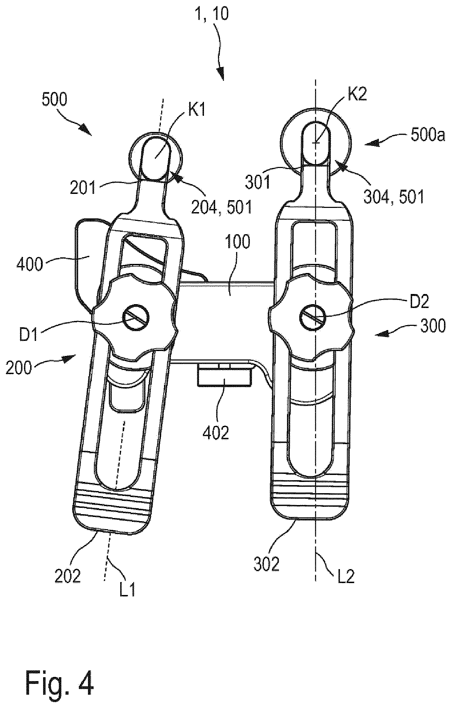

Gemäß den Fig. 1 bis 4 ist ein chirurgisches Instrument 1 zur Verwendung bei einer Kniegelenkersatzoperation vorgesehen und weist einen Grundkörper 100, einen ersten Taster 200, einen zweiten Taster 300 sowie wenigstens ein Kompensationselement 500 auf.

In der anhand Fig. 1 gezeigten Konfiguration ist das chirurgische Instrument 1 auf noch näher beschriebene Weise lösbar an einem Tibiaschnittblock 400 befestigt. An dem Tibiaschnittblock 400 ist ein extramedullärer Ausrichtstab 600 lösbar befestigt. Zusammen mit dem Tibiaschnittblock 400 und dem extramedullären Ausrichtstab 600 bildet das chirurgische Instrument 1 ein chirurgisches Instrumentensystem 10.

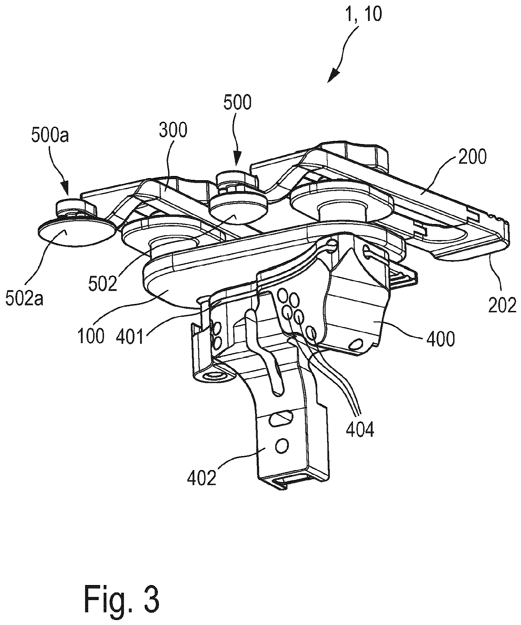

Der Tibiaschnittblock 400 weist einen Befestigungsabschnitt 402 auf. Der Befestigungsabschnitt 402 ist auf eine dem Fachmann bekannte Weise zur lösbaren Befestigung an dem extramedullären Ausrichtstab 600 eingerichtet. Der extramedulläre Ausrichtstab 600 weist zu diesem Zweck einen Befestigungsmechanismus 601 auf, welcher auf nicht näher gezeigte Weise mit dem Befestigungsabschnitt 402 zusammenwirkt. Weiter weist der Tibiaschnittblock 400 Durchgangsbohrungen 404 (Fig. 3) auf. Die Durchgangsbohrungen 404 erlauben eine Fixierung des Tibiaschnittblocks 400 an einer proximalen Tibia T. Hierfür werden nach erfolgter Ausrichtung des Tibiaschnittblocks 400 Befestigungsschrauben oder Pins durch die Durchgangsbohrungen 404 in die proximale Tibia T eingebracht.

Der Grundkörper 100 ist zur lösbaren Befestigung an dem Tibiaschnittblock 400 eingerichtet. Zu diesem Zweck weist der Grundkörper 100 bei der gezeigten Ausführungsform einen Befestigungsmechanismus 101 auf. Der Befestigungsmechanismus 101 wirkt auf nicht näher gezeigte Weise mit einem hierfür vorgesehenen Abschnitt des Tibiaschnittblocks 400 zusammen. Die Art und Weise der lösbaren Befestigung des Grundkörpers 100 an dem Tibiaschnittblock 400 ist im Hinblick auf die vorliegende Erfindung nicht von wesentlicher Bedeutung. Auf weitere diesbezügliche Details wird deshalb verzichtet.

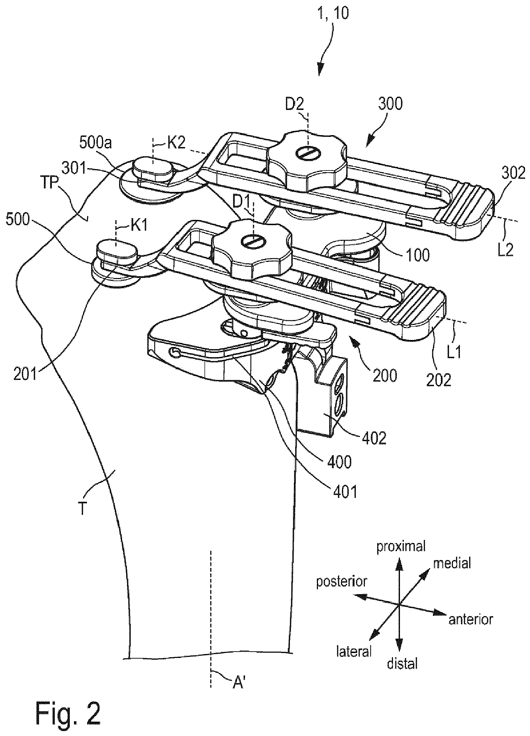

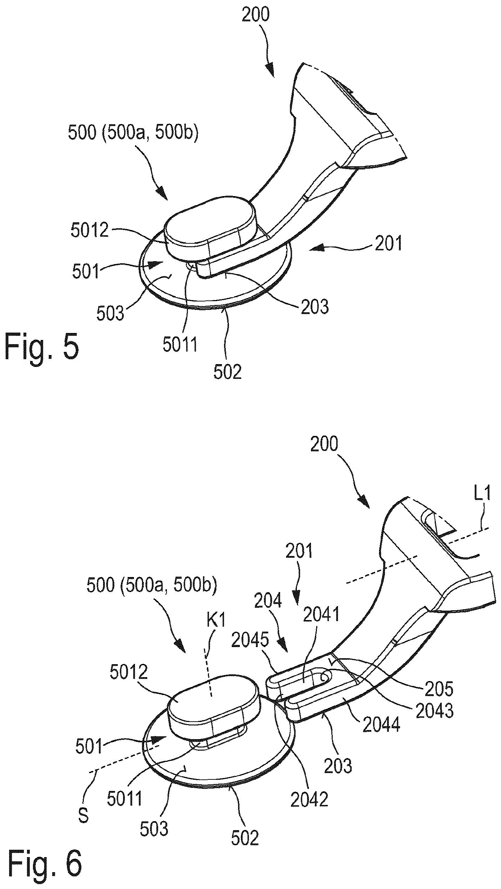

Der erste Taster 200 ist zwischen einem ersten Ende 201 und einem zweiten Ende 202 erstreckt. Der erste Taster 200 ist entlang einer ersten Längsachse L1 längserstreckt. Die erste Längsachse L1 definiert eine Haupterstreckungsrichtung des ersten Tasters 200. Der erste Taster 200 ist relativ zu dem Grundkörper 100 beweglich an demselben gelagert. Bei der gezeigten Ausführungsform ist der erste Taster um eine erste Schwenkachse D1 relativ zu dem Grundkörper 100 schwenkbeweglich. Zudem ist der erste Taster 200 entlang seiner ersten Längsachse L1 relativ zu dem Grundkörper 10 linearbeweglich geführt. Die konkrete Umsetzung der schwenkbeweglichen und der linearbeweglichen Führung des ersten Tasters 200 ist kein wesentlicher Aspekt der vorliegenden Erfindung. Auf weitere diesbezügliche Details kann deshalb verzichtet werden. Das erste Ende 201 des ersten Tasters 200 weist eine Referenzfläche 203 auf (siehe Fig. 5, 6). Die Referenzfläche 203 ist zur Kontaktierung eines Tibiaplateaus TP eingerichtet (siehe Fig. 2).

Der zweite Taster 300 ist zwischen einem ersten Ende 301 und einem zweiten Ende 302 längserstreckt. Der zweite Taster 300 weist eine zweite Längsachse L2 auf. Die zweite Längsachse L2 definiert eine Haupterstreckungsrichtung des zweiten Tasters 300. Der zweite Taster 300 ist um eine zweite Schwenkachse D2 relativ zu dem Grundkörper 100 schwenkbeweglich an demselben gelagert. Zudem ist der zweite Taster 300 entlang seiner Längsachse L2 relativ zu dem Grundkörper 100 linearbeweglich geführt. Das diesbezüglich zu dem ersten Taster 200 Gesagte gilt mutatis mutandis auch für den zweiten Taster 300. Die Art und Weise der schwenkbeweglichen und linearbeweglichen Lagerung des zweiten Tasters 300 in Relation zu dem Grundkörper 100 ist im Hinblick auf die vorliegende Erfindung nicht von wesentlicher Bedeutung. Weitere Details werden daher nicht erörtert. Das erste Ende 301 des zweiten Tasters 300 weist eine Referenzfläche 303 auf. Die Referenzfläche 303 des zweiten Tasters 300 ist zur Kontaktierung des Tibiaplateaus TP eingerichtet.

Das wenigstens eine Kompensationselement 500 ist zur wahlweisen Anbringung an einem der beiden Taster 200, 300 eingerichtet. In der anhand der Fig. 1 bis 4 gezeigten Konfiguration ist das Kompensationselement 500 an dem ersten Taster 200 angebracht. Das Kompensationselement 500 dient einem maßlichen Ausgleich etwaiger Knochen- und/oder Knorpelabnutzungen an dem Tibiaplateau TP. Dies auf noch näher beschriebene Weise. Das Kompensationselement 500 wird nachfolgend auch als erstes Kompensationselement bezeichnet. Weiter ist in der Konfiguration des chirurgischen Instruments 1 nach den Fig. 1 bis 4 ein zweites Kompensationselement 500a an dem zweiten Taster 300 angebracht. Dabei sind unterschiedliche Konfigurationen denkbar und vorgesehen. Beispielsweise kann das erste Kompensationselement 500 anstelle des zweiten Kompensationselements 500a an dem zweiten Taster angebracht sein und umgekehrt. Alternativ kann lediglich eines der Kompensationselemente 500, 500a an einem der beiden Taster 200, 300 angebracht sein. Weiter alternativ kann das chirurgische Instrument 1 auch ohne Anbringung der Kompensationselemente 500, 500a verwendet werden. Zur weiteren Erläuterung der Funktion und des Aufbaus des chirurgischen Instruments 1 wird nachfolgend zunächst auf eine solche Konfiguration ohne Kompensationselemente 500, 500a eingegangen. Dies unter Bezugnahme auf die in Fig. 2 dargestellte intraoperative Situation, in welcher das chirurgische Instrument 1 an dem Tibiaplateau TP der proximalen Tibia T platziert ist.

In der anhand Fig. 2 gezeigten Situation sind die beiden Schwenkachsen D1, D2 jeweils proximodistal ausgerichtet. Die erste Längsachse L1 und die zweite Längsachse L2 der beiden Taster 200, 300 sind in einer gemeinsamen Ebene (ohne Bezugszeichen) angeordnet. Die besagte Ebene ist mediolateral und anteroposterior erstreckt und insoweit eine Transversalebene. Die Referenzflächen 203, 303 der beiden Taster 200, 300 sind in einer gemeinsamen Referenzebene (ohne Bezugszeichen) angeordnet. Die Referenzebene ist mediolateral und anteroposterior erstreckt und folglich eine Transversalebene.

Ausgehend von der in Fig. 2 dargestellten Situation wird für die nachfolgende Erläuterung zunächst angenommen, dass weder das (erste) Kompensationselement 500 noch das zweite Kompensationselement 500a an den Tastern 200, 300 angebracht ist. In dieser Situation ist der Grundkörper 100 mittels des besagten Befestigungsmechanismus 101 lösbar an dem Tibiaschnittblock 400 befestigt. Der Tibiaschnittblock 400 weist eine dem Fachmann grundsätzlich bekannte Gestaltung und Funktion auf. Der Tibiaschnittblock 400 dient einer Schnittführung bei einer Resektion der proximalen Tibia T. Bei einer solchen Resektion wird das Tibiaplateau TP auf definierte Weise abgetrennt. Zu diesem Zweck weist der Tibiaschnittblock 400 einen Aufnahmeschlitz 401 auf, welcher zur Aufnahme und Führung eines Sägeblatts eingerichtet ist. Die Ausrichtung des Tibiaschnittblocks 400 - und damit auch diejenige des Aufnahmeschlitzes 401 - in Bezug auf anatomische Landmarken der proximalen Tibia T bestimmt die spätere Ausrichtung des nach der Resektion vorliegenden (künstlichen) Tibiaplateaus. Das künstliche Tibiaplateau legt wiederum die spätere Orientierung des künstlichen Kniegelenkersatzes und dessen Gelenkachse fest. Mittels des chirurgisches Instruments 1 kann der Tibiaschnittblock 400, genauer: dessen Aufnahmeschlitz 401, auf definierte Weise relativ zu der proximalen Tibia T ausgerichtet werden.

Diese Ausrichtung erfolgt zunächst unter Verwendung des (in Fig. 2 zeichnerisch ausgeblendeten) extramedullären Ausrichtstabs 600. Der extramedulläre Ausrichtstab 600 weist eine Längsachse A auf. Die Längsachse A wird entlang einer Längsachse A' (siehe Fig. 2) der Tibia T ausgerichtet. Die Längsachse A ist hiernach in Bezug auf die in den Fig. 1 und 2 eingezeichneten anatomischen Achsen proximodistal ausgerichtet. In einem weiteren Schritt werden die Taster 200, 300 relativ zu dem Tibiaplateau TP ausgerichtet.

Zur weiteren Erläuterung wird nachfolgend von einem ersten Zustand des Tibiaplateaus TP und einem zweiten Zustand des Tibiaplateaus TP ausgegangen. In dem ersten Zustand weist das Tibiaplateau TP keine oder keine praktisch erhebliche Abnutzung an seiner Knorpel- und/oder Knochenstruktur auf. In diesem ersten Zustand wird das chirurgische Instrument 1, wie bereits erläutert, ohne Kompensationselemente 500, 500a verwendet. Zur weiteren Ausrichtung werden sodann die Referenzflächen 203, 303 der beiden Taster 200, 300 mit dem Tibiaplateau TP kontaktiert. Der Kontakt zwischen den Referenzflächen 203, 303 und dem Tibiaplateau TP definiert eine proximodistale Position des Aufnahmeschlitzes 401. Zudem wird durch die Referenzierung an dem Tibiaplateau TP eine Rotation des Tibiaschnittblocks 400 um eine anteroposteriore Achse festgelegt. Die besagte Rotation legt eine Varus/Valgus-Ausrichtung des zu implantierenden künstlichen Kniegelenkersatzes fest.

Bei der gezeigten Ausführungsform sind die Referenzflächen 203, 303 jeweils plan. Bei in den Figuren nicht gezeigten Ausführungsformen sind die Referenzflächen hierzu unterschiedlich gestaltet. Beispielsweise können die Referenzflächen gewölbt, abgewinkelt oder auf sonstige Weise nicht-plan sein. Zudem können die Referenzflächen linien- und/oder punktförmig ausgebildet sein. Letzteres beispielsweise durch eine spitze Gestaltung der ersten Enden der beiden Taster.

Die Referenzflächen 203, 303 sind vorliegend - in der (gedachten) Konfiguration ohne Kompensationselemente - entlang ihrer jeweiligen Normalenrichtung an dem Tibiaplateau TP kontaktiert. Die erste Referenzfläche 203 ist entlang einer ersten Kontaktrichtung K1 an dem Tibiaplateau kontaktiert. Die zweite Referenzfläche 303 ist entlang einer zweiten Kontaktrichtung K2 an dem Tibiaplateau TP kontaktiert. Die beiden Kontaktrichtungen K1, K2 sind vorliegend parallel zueinander orientiert. Beide Kontaktrichtungen K1, K2 sind vorliegend proximodistal ausgerichtet.

Mittels der bereits erläuterten Relativbeweglichkeit der beiden Taster 200, 300 können die Referenzflächen 203, 303 anforderungsgerecht und präzise an dem Tibiaplateau TP positioniert werden.

Mit der vorbeschriebenen Referenzierung an dem Tibiaplateau TP wird der sogenannte Kinematic Alignment-Ansatz (KA) verfolgt. Bei diesem Ansatz besteht das Ziel darin, die Gelenkflächen des künstlichen Kniegelenkersatzes auf dem Niveau der präarthritischen, defektfreien natürlichen Gelenkflächen zu implantieren. Sofern Defekte vorliegen, können diese mittels des chirurgischen Instruments 1 auf einfache Weise kompensiert werden.

In dem anhand Fig. 2 gezeigten zweiten (defektbehafteten) Zustand des Tibiaplateaus TP erfolgt die Kompensation im Bereich der Referenzfläche 203 des ersten Tasters 200 mittels des ersten Kompensationselements 500. Im Bereich der Referenzfläche 303 des zweiten Tasters 300 erfolgt die Kompensation mittels des zweiten Kompensationselements 500a. Zur weiteren Erläuterung wird nachfolgend in erster Linie auf das erste Kompensationselement 500 und dessen Zusammenwirken mit dem ersten Taster 200 Bezug genommen. Das hierzu Offenbarte gilt mutatis mutandis auch im Hinblick auf das zweite Kompensationselement und dessen Zusammenwirken mit dem zweiten Taster 300.

Zur lösbaren Befestigung an dem ersten Taster 200 weist das Kompensationselement 500 einen Steckabschnitt 501 und eine Kontaktfläche 502 auf (Fig. 5 und 6). In angebrachtem Zustand des Kompensationselements 500 gelangt die Kontaktfläche 502 anstelle der Referenzfläche 203 an dem Tibiaplateau TP zur Anlage. Das erste Ende 201 weist einen komplementären Steckabschnitt 204 auf. Der Steckabschnitt 501 und der komplementäre Steckabschnitt 204 sind zur form- und/oder kraftschlüssigen lösbaren Verbindung miteinander eingerichtet. In miteinander verbundenem Zustand bilden der Steckabschnitt 501 und der komplementäre Steckabschnitt 204 eine lösbare Steckverbindung aus. Die Steckverbindung zwischen dem ersten Ende 201 und dem Kompensationselement 500 ist werkzeuglos herstellbar und lösbar. Das heißt ein ausführender Chirurg kann das Kompensationselement 500 rein manuell ohne Zuhilfenahme eines Werkzeugs an dem ersten Taster 200 anbringen und von diesem lösen.

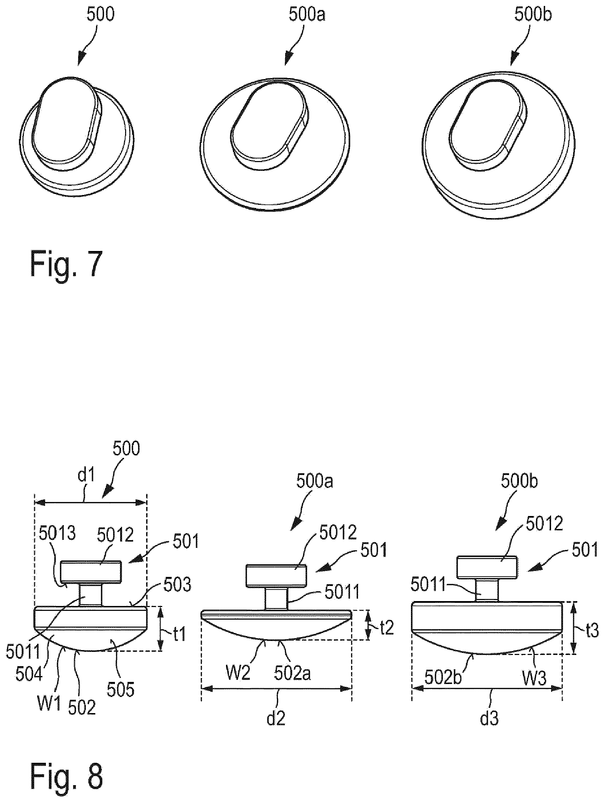

In angebrachtem Zustand des Kompensationselements (Fig. 5) ist die Kontaktfläche 502 in Bezug auf die erste Kontaktrichtung K1 unterhalb der Referenzfläche 203 angeordnet. Dabei ist die Kontaktfläche 202 um ein Dickenmaß t1 (siehe Fig. 8) entlang der ersten Kontaktrichtung K1 von der Referenzfläche 203 beabstandet. Das Dickenmaß t1 ist auf noch näher beschriebene Weise auf eine Ausprägung des zu kompensierenden Defekts abgestimmt.

Die besagte Steckverbindung weist bei der gezeigten Ausführungsform eine Steckrichtung S auf, welche parallel zu der ersten Längsachse L1 des ersten Tasters 200 und zudem orthogonal zu der ersten Kontaktrichtung K1 orientiert ist. Die Steckrichtung S kann auch als Steckachse bezeichnet werden. In der Verwendung des chirurgischen Instruments 1 ist die Steckrichtung S in einer nicht näher bezeichneten Transversalebene angeordnet.

Bei der gezeigten Ausführungsform weist der komplementäre Steckabschnitt 204 einen längserstreckten Aufnahmeschlitz 2041 auf. Der Aufnahmeschlitz 2041 ist zur kraft- und/oder formschlüssigen Aufnahme des Steckabschnitts 501 eingerichtet. Eine Längsachse (ohne Bezugszeichen) des Aufnahmeschlitzes 2041 ist parallel zu der ersten Längsachse L1 des ersten Tasters 200 orientiert. Die Längserstreckung des Aufnahmeschlitzes 2041 definiert vorliegend die Steckrichtung S. Der Aufnahmeschlitz 2041 ist gerade längserstreckt.

Bei der gezeigten Ausführungsform weist der Aufnahmeschlitz 2041 einends eine Einführöffnung 2042 und andernends einen Anschlag 2043 auf. Der Steckabschnitt 501 des Kompensationselements 500 kann durch die Einführöffnung 2042 in den Aufnahmeschlitz 2041 eingeführt werden. Der Anschlag 2043 dient einer formschlüssigen Begrenzung der Einführtiefe.

Der Aufnahmeschlitz 2041 reicht ausgehend von der Referenzfläche 203 bis auf eine Rückseite 205 des ersten Endes 201. Die Rückseite 205 liegt der Referenzfläche 203 entlang der ersten Kontaktrichtung K1 gegenüber. Vorliegend ist die Rückseite 205 plan. Eine Normalenrichtung der Rückseite 205 ist in der Verwendung des chirurgischen Instruments 1 proximal orientiert. Folglich ist der Aufnahmeschlitz 2041 entlang seiner Hochrichtung durchgängig zwischen der Referenzfläche 203 und der Rückseite 205. Bei einer in den Figuren nicht gezeigten Ausführungsform ist der Aufnahmeschlitz 2041 stattdessen in Hochrichtung einseits begrenzt und nach Art einer Nut ausgeführt. Die Durchgängigkeit des Aufnahmeschlitzes 2041 bietet - in einem nicht angebrachten Zustand des Kompensationselements 500 - eine verbesserte Einsehbarkeit des Tibiaplateaus TP. Hierdurch kann der Chirurg in verbesserter Weise visuell kontrollieren, ob die Referenzfläche 203 das Tibiaplateau TP kontaktiert.

Bei der gezeigten Ausführungsform ist der Aufnahmeschlitz 2041 zwischen zwei Zinkenabschnitten 2044, 2045 gebildet. Die Zinkenabschnitte 2044, 2045 können auch als erster Zinkenabschnitt 2044 und zweiter Zinkenabschnitt 2045 bezeichnet werden. Die beiden Zinkenabschnitte 2044, 2045 liegen einander entlang einer Querrichtung des Aufnahmeschlitzes 2041 gegenüber. Die Zinkenabschnitte 2044, 2045 sind jeweils parallel zu der ersten Längsachse L1 längserstreckt. Nicht näher bezeichnete Stirnendabschnitte der Zinkenabschnitte 2044, 2045 beranden die Einführöffnung 2042. Infolge der Zinkenabschnitte 2044, 2045 weist das erste Ende 201 vorliegend eine gegabelte Form oder auch Gabelform auf. Die Zinkenabschnitte 2044, 2045 sind in Querrichtung des Aufnahmeschlitzes 2041 nach Art einer Biegefeder elastisch federbeweglich. Die Federbeweglichkeit ist bei unterschiedlichen Ausgestaltungen unterschiedlich stark ausgeprägt. Bei einer Ausgestaltung ist keine oder keine praktisch erhebliche Federbeweglichkeit vorhanden.

Es versteht sich, dass der zweite Taster 300 an seinem ersten Ende 301 ebenfalls einen komplementären Steckabschnitt 304 aufweist. Das zu dem komplementären Steckabschnitt 204 des ersten Tasters 200 Gesagte gilt mutatis mutandis auch für den komplementären Steckabschnitt 304. Vorliegend ist der komplementäre Steckabschnitt 304 des zweiten Tasters 300 identisch zu dem komplementären Steckabschnitt 204 des ersten Tasters 200.

Der Steckabschnitt 501 des Kompensationselements 500 liegt der Kontaktfläche 502 entlang der ersten Kontaktrichtung K1 gegenüber. Der Steckabschnitt 501 ragt von einer Rückseite 503 des Kompensationselements auf. Der Steckabschnitt 501 ragt entlang der ersten Kontaktrichtung K1 von der Rückseite 503 auf. Die Rückseite 503 ist bei der gezeigten Ausführungsform plan. Eine Normalenrichtung (ohne Bezugszeichen) der Rückseite 503 ist parallel zu der ersten Kontaktrichtung K1 ausgerichtet und damit in der Verwendung des chirurgischen Instruments 1 proximal orientiert (siehe Fig. 2).

Bei der gezeigten Ausführungsform weist der Steckabschnitt 501 einen Schaft 5011 und einen Kopf 5012 auf. Der Schaft 5011 grenzt einends an die Rückseite 503 und andernends an den Kopf 5012. Der Schaft 5011 ist entlang der ersten Kontaktrichtung K1 längserstreckt. Der Schaft 5011 ist maßlich auf den Aufnahmeschlitz 2041 abgestimmt und umgekehrt. In angebrachtem Zustand des Kompensationselements 500 ist der Schaft 5011 kraft- und/oder formschlüssig in dem Aufnahmeschlitz 2041 gehalten. Die Längserstreckung des Schafts 5011 ist orthogonal zu der Steckrichtung S. Folglich wird der Schaft 5011 quer zu seiner Längserstreckung in den Aufnahmeschlitz 2041 eingeführt und aus diesem herausgenommen.

Der Kopf 5012 ist fest mit dem Schaft 5011 verbunden und weist einen nicht näher bezeichneten Durchmesser auf, welcher größer ist als ein Durchmesser des Schafts 5011. Mittels des Kopfs 5012 ist der Schaft 5011 entlang der ersten Kontaktrichtung K1 distal formschlüssig an dem ersten Ende 201 gehalten. Proximal ist der Schaft 5011 mittels der Rückseite 503 formschlüssig an dem ersten Ende 201 gehalten. In angebrachtem Zustand des Kompensationselements 500 liegt folglich die Rückseite 503 an der Referenzfläche 203 an. Eine Vorderseite 5013 des Kopfs 5012 (siehe Fig. 8) liegt an der Rückseite 205 des ersten Endes 201 an.

Bei der gezeigten Ausführungsform weist der Schaft 5011 einen nicht näher gezeigten rotationsunsymmetrischen Querschnitt auf. Die Form des rotationsunsymmetrischen Querschnitts des Schafts 5011 entspricht der in den Figuren gezeigten Form der Stirnfläche des Kopfs 5012. Die rotationsunsymmetrische Querschnittsform des Schafts 5011 wirkt einer ungewollten Rotation des Kompensationselements 500 relativ zu dem ersten Ende 201 entgegen.

Die Kontaktfläche 202 ist bei der gezeigten Ausführungsform konvex. Die konvexe Wölbung der Kontaktfläche 502 erlaubt eine verbesserte visuelle Kontrolle des Kontakts zwischen Kompensationselement 500 und Tibiaplateau TP.

Das Kompensationselement 500 weist bei der gezeigten Ausführungsform einen Kugelsegmentabschnitt 504 mit einer Kugelkalotte 505 auf. Die Kugelkalotte 505 bildet die Kontaktfläche 502.

Wie anhand der Fig. 7 gezeigt ist, weist das chirurgische Instrument 1 bei der gezeigten Ausführungsform mehrere unterschiedliche Kompensationselemente 500, 500a, 500b auf. Die Kompensationselemente 500, 500a, 500b werden nachfolgend auch als erstes Kompensationselement 500, zweites Kompensationselement 500a und drittes Kompensationselement 500b bezeichnet. Die unterschiedlichen Kompensationselemente 500, 500a, 500b erlauben eine Kompensation unterschiedlich stark ausgeprägter Defekte. Die Kompensationselemente 500, 500a, 500b unterscheiden sich vorliegend im Hinblick auf ihr jeweiliges Dickenmaß t1, t2, t3 (siehe Fig. 8). Das erste Kompensationselement 500 weist ein erstes Dickenmaß t1 auf. Das zweite Kompensationselement 500a weist ein zweites Dickenmaß t2 auf. Das dritte Kompensationselement 500b weist ein drittes Dickenmaß t3 auf. Vorliegend ist das zweite Dickenmaß t2 kleiner als das erste Dickenmaß t1 und kleiner als das dritte Dickenmaß t3. Das erste Dickenmaß t1 ist größer als das zweite Dickenmaß t2 und kleiner als das dritte Dickenmaß t3. Das dritte Dickenmaß t3 ist größer als das erste Dickenmaß t1 und größer als das zweite Dickenmaß t2. Je nachdem, ob ein vergleichsweise schwach oder vergleichsweise stark ausgeprägter Defekt an dem Tibiaplateau TP vorhanden ist, wählt der Chirurg ein vergleichsweise kleines oder vergleichsweise großes der unterschiedlichen Dickenmaße t1, t2, t3 aus.

Die unterschiedlichen Kompensationselemente 500, 500a, 500b sind hinsichtlich der Gestaltung des jeweiligen Steckabschnitts identisch. Folglich weist jedes der unterschiedlichen Kompensationselemente 500, 500a, 500b vorliegend einen Schaft 5011 und einen Kopf 5012 auf. Die identische Gestaltung der Steckabschnitte 501 an den unterschiedlichen Kompensationselementen 500, 500a, 500b gewährleistet deren Austauschbarkeit. Mit anderen Worten kann jedes der Kompensationselemente 500, 500a, 500b erforderlichenfalls an dem ersten Ende 201 des ersten Tasters 200 oder an dem ersten Ende 301 des zweiten Tasters 300 angebracht werden.

Weiter weisen die unterschiedlichen Kompensationselemente 500, 500a, 500b vorliegend unterschiedliche Durchmesser d1, d2, d3 der jeweiligen Kontaktflächen 502, 502a, 502b auf. Zudem weisen die Kontaktflächen 502, 502a, 502b unterschiedliche Wölbungen W1, W2, W3 auf. Im Speziellen weist das erste Kompensationselement einen ersten Durchmesser d1 auf. Das zweite Kompensationselement 500a weist einen zweiten Durchmesser d2 auf. Das dritte Kompensationselement 500b weist einen dritten Durchmesser d3 auf. Die Durchmesser d1, d2, d3 können auch als Außendurchmesser (der Kontaktfläche) bezeichnet werden. Vorliegend ist der erste Durchmesser d1 kleiner als der zweite Durchmesser d2 und kleiner als der dritte Durchmesser d3. Der zweite Durchmesser d2 ist größer als der erste Durchmesser d1 und geringfügig kleiner als der dritte Durchmesser d3. Der dritte Durchmesser d3 ist größer als der erste Durchmesser d1 und geringfügig größer als der zweite Durchmesser d2.

Es versteht sich, dass die vorliegende Anzahl der unterschiedlichen Kompensationselemente 500, 500a, 500b als rein exemplarisch zu verstehen ist. Selbstverständlich kann das chirurgische Instrument 1 in unterschiedlichen Ausgestaltungen mehr oder weniger als die vorliegend gezeigten drei unterschiedlichen Kompensationselemente 500, 500a, 500b aufweisen. Bei einer Ausgestaltung ist lediglich ein einziges Kompensationselement vorhanden.

Bei einer weiteren Ausgestaltung ist eine Vielzahl unterschiedlicher Kompensationselemente, beispielsweise zehn, zwanzig oder dreißig, vorhanden.

## Patentansprüche

1. Chirurgisches Instrument (1) zur Verwendung bei einer Kniegelenkersatzoperation, aufweisend
einen Grundkörper (100), welcher eingerichtet ist zur lösbaren Befestigung an einem Tibiaschnittblock (400) zur Schnittführung an einer proximalen Tibia (T),
einen ersten Taster (200) und einen zweiten Taster (300), welche jeweils relativ zu dem Grundkörper (100) beweglich an demselben gelagert und zwischen einem ersten Ende (201, 301) und einem zweiten Ende (202, 302) längserstreckt sind,
wobei die ersten Enden (201, 301) jeweils eine Referenzfläche (203, 303) aufweisen, welche zur Kontaktierung eines Tibiaplateaus (TP) der Tibia (T) entlang einer Kontaktrichtung (K1, K2) eingerichtet ist,
wobei wenigstens ein zur wahlweisen Anbringung an einem der beiden Taster (200, 300) vorgesehenes Kompensationselement (500) vorhanden ist und einen Steckabschnitt (501) sowie eine Kontaktfläche (502) aufweist, und wobei die ersten Enden (201, 301) jeweils einen komplementären Steckabschnitt (204, 304) aufweisen, welcher zur form- und/oder kraftschlüssigen lösbaren Verbindung mit dem Steckabschnitt (501) des Kompensationselements (500) eingerichtet ist, und wobei die Kontaktfläche (502) - in einem an dem jeweiligen Taster (200, 300) angebrachten Zustand des Kompensationselements (500) - zur Kontaktierung des Tibiaplateaus (TP) anstelle der jeweiligen Referenzfläche (203, 303) eingerichtet und entlang der Kontaktrichtung (K1, K2) um ein Dickenmaß (t1) beabstandet von der jeweiligen Referenzfläche (203, 303) angeordnet ist.

2. Chirurgisches Instrument (1) nach Anspruch 1, **dadurch gekennzeichnet, dass** der Steckabschnitt (501) entlang einer Steckrichtung (S) mit dem jeweiligen komplementären Steckabschnitt (204, 304) zusammensteckbar ist, welche parallel zu einer Längsachse (L1, L2) des jeweiligen Tasters (200, 300) und/oder orthogonal zu der Kontaktrichtung (K1, K2) orientiert ist.

3. Chirurgisches Instrument (1) nach Anspruch 1 oder 2, **dadurch gekennzeichnet, dass** der Steckabschnitt (501) und/oder die komplementären Steckabschnitte (204, 304) jeweils einen rotationsunsymmetrischen Querschnitt aufweisen.

4. Chirurgisches Instrument (1) nach einem der vorhergehenden Ansprüche, **dadurch gekennzeichnet, dass** die komplementären Steckabschnitte (204, 304) jeweils einen längserstreckten Aufnahmeschlitz (2041) aufweisen, welcher zur kraft- und/oder formschlüssigen Aufnahme des Steckabschnitts (501) eingerichtet ist.

5. Chirurgisches Instrument (1) nach Anspruch 4, **dadurch gekennzeichnet, dass** der Aufnahmeschlitz (2041) entlang seiner Längsrichtung zwischen einer Einführöffnung (2042) und einem Anschlag (2043) längserstreckt ist.

6. Chirurgisches Instrument (1) nach Anspruch 4 oder 5, **dadurch gekennzeichnet, dass** der Aufnahmeschlitz (2041) in einer quer zu seiner Längsrichtung orientierten Hochrichtung von der Referenzfläche (203) bis auf eine Rückseite (205) des jeweiligen ersten Endes (201, 301) reicht, wobei die Rückseite (205) der Referenzfläche (203) entlang der Kontaktrichtung (K1, K2) gegenüberliegt.

7. Chirurgisches Instrument (1) nach einem der Ansprüche 4 bis 6, **dadurch gekennzeichnet, dass** der Aufnahmeschlitz (2041) zwischen zwei, vorzugsweise federelastischen, Zinkenabschnitten (2044, 2045) des jeweiligen ersten Endes (201, 301) gebildet ist, wobei die Zinkenabschnitte (2044, 2045) in einer quer zu der Längsrichtung des Aufnahmeschlitzes (2041) orientierten Querrichtung einander gegenüberliegen.

8. Chirurgisches Instrument (1) nach einem der vorhergehenden Ansprüche, **dadurch gekennzeichnet, dass** der Steckabschnitt (501) von einer Rückseite (503) des Kompensationselements (500) aufragt, welche der Kontaktfläche (502) entlang der Kontaktrichtung (K1, K2) gegenüberliegt.

9. Chirurgisches Instrument (1) nach Anspruch 8, **dadurch gekennzeichnet, dass** der Steckabschnitt einen Schaft (5011) und einen Kopf (5012) aufweist, wobei der Schaft (5011) zwischen der Rückseite (503) des Kompensationselements (500) und dem Kopf (5012) längserstreckt und quer zu seiner Längserstreckung mit dem komplementären Steckabschnitt (204, 304) des jeweiligen ersten Endes (201, 301) zusammensteckbar ist, wobei der Kopf (5012) einen größeren Durchmesser als der Schaft (5011) aufweist.

10. Chirurgisches Instrument (1) nach Anspruch 9, **dadurch gekennzeichnet, dass** der Schaft (5011) und/oder der Kopf (5012) einen rotationsunsymmetrischen Querschnitt aufweisen.

11. Chirurgisches Instrument (1) nach einem der vorhergehenden Ansprüche, **dadurch gekennzeichnet, dass** die Kontaktfläche (502) konvex gewölbt ist.

12. Chirurgisches Instrument (1) nach einem der vorhergehenden Ansprüche, **dadurch gekennzeichnet, dass** das wenigstens eine Kompensationselement (500) einen Kugelsegmentabschnitt (504) mit einer Kugelkalotte (505) aufweist, welche die Kontaktfläche (502) bildet.

13. Chirurgisches Instrument (1) nach einem der vorhergehenden Ansprüche, **dadurch gekennzeichnet, dass** mehrere unterschiedliche Kompensationselemente (500, 500a, 500b) zur wahlweisen Anbringung an den beiden Tastern (200, 300) vorhanden sind, wobei die unterschiedlichen Kompensationselemente (500, 500a, 500b) sich, vorzugsweise ausschließlich, unterscheiden im Hinblick auf ihr jeweiliges Dickenmaß (t1, t2, t3) und/oder einen Durchmesser (d1, d2, d3) der jeweiligen Kontaktfläche (502, 502a, 502b) und/oder eine Wölbung (W1, W2, W3) der jeweiligen Kontaktfläche (502, 502a, 502b).

14. Chirurgisches Instrumentensystem (10) zur Verwendung bei einer Kniegelenkersatzoperation mit einem chirurgischen Instrument (1) nach einem der vorhergehenden Ansprüche, einem Tibiaschnittblock (400), an welchem das chirurgische Instrument (1) lösbar befestigt ist, und mit einem extramedullären Ausrichtstab (600), welcher lösbar an dem Tibiaschnittblock (400) befestigt ist.
